# EUROPEAN PATENT APPLICATION

(11) **EP 4 772 694 A1**
(43) Date of publication of application: **08.07.2026**
(21) Application number: 25221072.9
(22) Date of filing: 05.12.2025
(51) Int. Cl.: D21H 13/10, C12N 1/14, D21H 13/30, D21H 27/10, B32B 33/00, C12N 1/00

(54) **METHOD FOR PREPARING JANUS PAPER FROM MYCELIA AND JANUS PAPER**

(30) Priority: 05.12.2024 CN 202411776477
(71) Applicant: Jilin Agricultural University, Changchun, Jilin 130118 (CN)
(72) Inventor: XIAO, Shijun, Changchun City, 130118 (CN); HAN, Xuerong, Changchun City, 130118 (CN); YU, Zhenyang, Changchun City, 130118 (CN); LI, Yu, Changchun City, 130118 (CN)
(74) Representative: Patent 42

(57) **Abstract**

A method for preparing Janus paper by mycelia and Janus paper. The method includes the steps of: step 1, inactivation of mycelia; step 2, fibrillation of mycelia; step 3, purification of solid-state fermented mycelia; step 4, gelation of secondary fermented mycelia; and step 5, preparation of Janus paper. In the present disclosure, Janus paper is prepared using edible fungus mycelia, which can replace the traditional papermaking method that uses wood as a main raw material. No plastics or chemical coatings need to be added, and this disclosure has the advantages of environmental protection, low carbon emissions, and biodegradability.

## Description

### TECHNICAL FIELD

The present disclosure relates to the technical field of polymer compounds, and in particular to a method for preparing Janus paper by mycelia and Janus paper.

### BACKGROUND

With the implementation of plastic restrictions and plastic bans in various countries, many industries such as production, retail enterprises and catering have begun to use paper packaging instead of plastic packaging in actual production and sales. However, the heavy use of paper can lead to a serious depletion of natural resources.

Silicone oil paper is a commonly used packaging paper in food industry, which is mainly included by three layers, namely backing paper, coated paper and silicone oil. It has the characteristics of high temperature resistance, oil resistance and moisture resistance. Therefore, it is widely used in the packaging of fried, baked, meat and other foods, and generally comes into direct contact with food. The component of silicone oil is cyclic polydimethylsiloxane. Although silicone oil is allowed to be added to food packaging materials in China, silicone oil is soluble in oily environment, especially unqualified silicone oil paper, which may release chemicals harmful to human body at high temperatures, causing threats to human health. In addition, the coating of silicone oil paper is to coat plastic on the surface of the paper through a casting machine. The coating is divided into food grade and industrial grade, and the cost of food grade coated paper is relatively high.

In terms of environment, the production of silicone oil paper will consume forest resources, and the waste after the use of silicone oil paper has become a difficult problem in environmental management. Silicone oil paper includes plastic coating, silicone oil coating and other components. These components are difficult to degrade in the natural environment and accumulate over time, which will eventually hinder the air permeability and water permeability of the soil, and even pollute groundwater. Silicone oil paper is not only difficult to degrade, but also very difficult to recycle. In the recycling process of silicone oil paper, different materials such as backing paper, coating and silicone oil coating need to be separated first. However, the separation process of these materials is complicated and costly, but the recycling efficiency is low. Therefore, silicone oil paper is mainly used as disposable paper in many industrial fields. After being discarded, the silicone oil paper cannot be recycled, and it is difficult to degrade, which is harmful to the environment. Therefore, the development of environmentally friendly, degradable and food-grade packaging paper prepared from natural biomaterials is of great significance to food safety, environmental protection and consumer health.

In nature, fungi, as the decomposer of waste, maintain ecological balance with eukaryotes such as plants and animals. Among the fungi, there are about 2,000 kinds of edible fungi known to human beings at present. Mycelium is the main component of edible fungi; and unlike fruiting bodies that grow above ground, mycelium grows underground and is included by long filamentous substances. In recent years, edible fungi have been widely used in fields such as food, medicine, and industrial materials due to their abundant nutrition, significant efficacy, and excellent properties. However, the yield of wild edible fungi is small and difficult to obtain, and the artificial cultivation cycle of edible fungi is very long. For example, the artificial cultivation cycle of Ganoderma lucidum, precious edible fungi in China, is more than 6 months, which not only increases the cultivation cost of edible fungi, but also limits its application in industrial materials. As a perfect substitute for edible fungi, mycelium not only includes nutritional value almost equivalent to that of edible fungi, but also has good strength and toughness, and shows good application potential in the manufacture of various industrial products. Compared with fruiting bodies, the cultivation cycle of mycelium is short, generally only about 10 days. Therefore, this greatly reduces the production cost and broadens the application prospect of mycelium as an industrial material. Applying the excellent material properties of mycelium to paper manufacturing will greatly reduce the consumption of forests and dependence on raw wood in the paper industry.

### SUMMARY

The present disclosure provides a method for preparing Janus paper by mycelia and Janus paper to solve the problems of resource waste and large pollution in related paper-making technology.

According to one aspect of the present disclosure, a method for preparing Janus paper by mycelia is provided, the mycelium being edible fungus mycelium, includes the steps of:
step 1, mycelium inactivation: placing solid-state fermented mycelia and secondary fermented mycelia in drying ovens for drying to obtain inactivated solid-state fermented mycelia and inactivated secondary fermented mycelia; and the solid-state fermented mycelia referring to edible fungus mycelia cultured via solid-state fermentation, the secondary fermented mycelia referring to edible fungus mycelia processed via secondary fermentation, the solid-state fermentation referring to a process of culturing edible fungus mycelia using solid media, the secondary fermentation referring to a process of decomposing edible fungus mycelia using another microorganism, and the edible fungi referring to edible mushrooms;
step 2, fibrillation of mycelia: crushing the inactivated solid-state fermented mycelia and inactivated secondary fermented mycelia obtained in step 1 in crushers; preparing the crushed mycelia into Suspension A and Suspension B with water, and grinding Suspension A and Suspension B; and performing centrifugation on the ground Suspension A and Suspension B using centrifuges to separate fibrillated solid-state fermented mycelia and fibrillated secondary fermented mycelia;
step 3, purification of solid-state fermented mycelia: preparing the fibrillated solid-state fermented mycelia obtained in step 2 into Suspension C with water; performing centrifugation on Suspension C using a centrifuge to obtain solid-state fermented mycelial precipitate; placing the solid-state fermented mycelial precipitate in a sodium hydroxide solution, and stirring a mixture of the precipitate and sodium hydroxide in a constant-temperature water bath; and repeatedly adding water to the mixture and performing centrifugation to obtain a precipitate as purified solid-state fermented mycelia;
step 4, gelation of secondary fermented mycelia: preparing the fibrillated secondary fermented mycelia obtained in step 2 into Suspension D with water; treating Suspension D using a homogenizer to make Suspension D more uniform; placing Suspension D in a constant-temperature water bath, adding epigallocatechin gallate (EGCG), and stirring; and performing centrifugation on Suspension D using a centrifuge after stirring to obtain a precipitate as gelled secondary fermented mycelia; and
step 5, preparation of Janus paper: preparing the purified solid-state fermented mycelia obtained in step 3 and the gelled secondary fermented mycelia obtained in step 4 into Suspension E and Suspension F with water; pouring Suspension E and Suspension F into paper forming molds in sequence to form mycelial filter cakes; pressing the two overlapping mycelial filter cakes using a paper flattening machine; applying a uniformly distributed weight on the forming mold with the two filter cakes; placing the forming mold with the filter cakes and the weight into an oven for drying; taking out the mold with the two types of mycelia and the weight after drying, and cooling the mold with the two types of mycelia and the weight at room temperature; and removing the weight and the mold after cooling to obtain Janus paper prepared from edible fungus mycelia.

Alternatively, in step 3, a concentration of sodium hydroxide solution ranges from 0.5 mol/L to 1.5 mol/L, and a concentration of solid-state fermented mycelial precipitate in the sodium hydroxide solution ranges from 30 g/100 mL to 50 g/100 mL.

Alternatively, in step 3, the stirring mixtures of the precipitates and sodium hydroxide in constant-temperature water baths; and repeatedly adding water to the mixtures and performing centrifugation to obtain precipitates as purified solid-state fermented mycelia includes the steps of: placing the mixture in a constant-temperature water bath at 50-65°C, and stirring the mixture with an electric stirrer at a rotation speed of 220-420 r/min for 1.5-3.5 hours; and adding purified water of 2.5-3.5 times the volume to the mixture, performing centrifugation at a rotation speed of 6500-8500 r/min for 15-20 minutes, collecting the precipitate, and repeating operations of adding water to the precipitate and centrifuging for 3-4 times to obtain the precipitate as purified solid-state fermented mycelia.

Alternatively, in step 4, a weight ratio of EGCG to the secondary fermented mycelia ranges from 1:50 to 1:6, a weight ratio of EGCG to a total weight of mycelia ranges from 1:100 to 1:12, and the total weight of mycelia refers to a total weight of the secondary fermented mycelia and the solid-state fermented mycelia.

Alternatively, the step 4 includes the steps of: preparing the fibrillated secondary fermented mycelia obtained in step 2 into Suspension D with a concentration of 30-50 g/100 mL using purified water, and treating Suspension D with a homogenizer at a rotation speed of 16900-21900 r/min for 1-4 minutes; placing Suspension D in a constant-temperature water bath at 75-95°C, adding EGCG to Suspension D, with a concentration of EGCG in Suspension D being 1-5 g/100 mL, and stirring with an electric stirrer at a rotation speed of 200-400 r/min for 30-60 minutes; and performing centrifugation on Suspension D using a centrifuge, setting a rotation speed to 6500-8500 r/min and centrifuging for 15-20 minutes to obtain the precipitate as gelled secondary fermented mycelia.

Alternatively, after step 2, the method further includes the steps of: screening particle sizes of fibrillated solid-state fermented mycelia and fibrillated secondary fermented mycelia using a laser particle size analyzer, ensuring both are no more than 200 µm; and screening fibrillation rates of fibrillated solid-state fermented mycelia and fibrillated secondary fermented mycelia using a fiber morphology analyzer, ensuring both are no less than 50%.

Alternatively, after step 3, the method further includes the steps of: screening the pH value of purified solid-state fermented mycelia using a pH meter, ensuring the pH is 7.5±0.5; screening the average length of purified solid-state fermented mycelia using a laser confocal fluorescence microscope, ensuring the average length is no more than 20 µm; and screening the heat resistance of purified solid-state fermented mycelia using an oven, ensuring the heat resistance is no less than 180°C.

Alternatively, after step 4, the method further includes the steps of: screening the gelled secondary fermented mycelia using a scanning electron microscope to ensure the gelled secondary fermented mycelia exhibit a network structure; screening the gelled secondary fermented mycelia using a rheometer to ensure the viscous modulus is no less than 35 Pa and elastic modulus is no less than 40 Pa; and screening the gelled secondary fermented mycelia using a texture analyzer to ensure the hardness is no less than 350 g/cm².

Alternatively, after step 5, the method further includes the steps of: screening a solid-state fermented mycelium paper with a relatively rough surface and an oil-phase interface contact angle not exceeding 75°; screening an oil-phase interface contact angle of a secondary fermented mycelium paper surface to ensure the angle ranges from 90° to 180°; and screening Janus paper to ensure a compressive strength is no less than 3 MPa, transverse tear strength no less than 1500 mN, longitudinal tear strength no less than 1200 mN, transverse tensile strength no less than 4.5 kN/m, longitudinal tensile strength no less than 5.5 kN/m, and elongation at break no less than 15%.

According to another aspect of the present disclosure, there is also provided a Janus paper prepared from the method for preparing Janus paper by mycelia described above.

The present disclosure has the following advantageous effects.

The present disclosure can replace the traditional manufacturing method of paper with wood, plastic film coating, chemical coating and the like as the main raw materials, does not need to cut down forests, does not depend on logs, and can protect forest resources. Janus paper is made from edible fungus mycelium, which is natural, green, environmentally friendly and low-carbon, and does not add any chemical raw materials such as chemical coating, plasticizer, binder, cross-linking agent, bleaching agent and fluorescent agent. It is non-toxic to human body and environmentally friendly, and can directly contact with food, reaching food grade, and has simple process and low cost. In terms of performance, Janus paper has two different properties, that is, one side of the paper absorbs oil and the other side does not leak oil. It has good oil absorption, heat resistance, compressive strength, tensile strength and tear strength, etc. It is suitable for packaging paper and has good application potential in industrial fields such as food and medicine, as well as in service fields such as express delivery and logistics.

### BRIEF DESCRIPTION OF THE DRAWINGS

The accompanying drawings described herein are intended to provide a further understanding of the present disclosure and form a part of the present disclosure, and the schematic examples of the present disclosure and the description thereof are intended to explain the present disclosure, and do not constitute an unduly limiting of the present disclosure. In the accompanying drawings:
FIG. 1 is a schematic flow diagram of a method for preparing Janus paper by mycelia according to an example of the present disclosure.
FIG. 2 is a schematic flow diagram in an implementation scenario according to an example of the present disclosure.
FIG. 3 shows a microscopic morphology of solid-state fermented mycelia.
FIG. 4 shows a microscopic morphology of a network structure of gelled secondary fermented mycelia.
FIG. 5 is a molecular structure diagram of a solid-state fermented mycelium paper.
FIG. 6 is a molecular structure diagram of a secondary fermentation mycelium paper.
FIG. 7 is a schematic diagram of a morphology of a prepared Janus paper.

### DETAILED DESCRIPTION

For those skilled in the art to better understand the solutions of the present disclosure, technical solutions in the examples of the present disclosure will be described clearly and completely in the following with reference to the attached drawings in the examples of the present disclosure. Obviously, all the described examples are only some, rather than all examples of the present disclosure. Based on the examples in the present disclosure, all other examples obtained by those ordinary skilled in the art without creative efforts belong to the protection scope of the present disclosure. It is noted that the examples and features of the examples in the present disclosure can be combined with each other without conflict.

Moreover, reference herein to "one example" or "an example" refers to a particular feature, structure, or characteristic that may be included in at least one implementation of the present disclosure. The appearances of "in one example" in different places in this specification do not all refer to the same example, nor are they separate or alternative examples that are mutually exclusive of other examples.

Furthermore, the terms "including" and "having" and any variations thereof are intended to cover non-exclusive inclusion. For example, a process, method, product or equipment that includes a series of steps or units need not be limited to those explicitly listed, but may include other steps or units that are not explicitly listed or inherent to these processes, methods, products or equipment.

### Example 1

An example of the present disclosure provides a method for preparing Janus paper by mycelia, which adopts edible fungus mycelium as a main raw material for preparing paper.

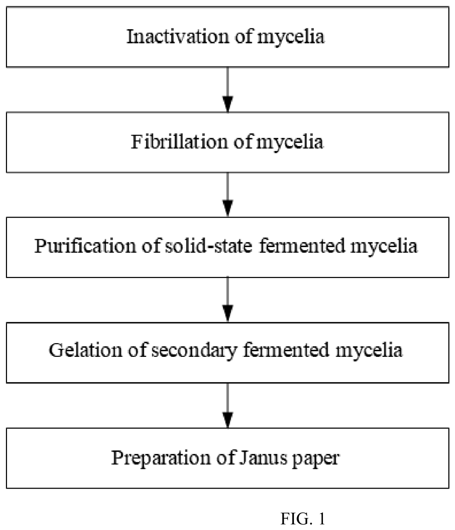

Referring to FIG. 1, FIG. 1 is a schematic flow diagram of a method for preparing Janus paper by mycelia according to an example of the present disclosure, and as shown in FIG. 1, the method includes the following steps.

In step 1, inactivation of mycelia: solid-state fermented mycelia and secondary fermented mycelia were placed in drying ovens for drying to obtain inactivated solid-state fermented mycelia and inactivated secondary fermented mycelia.

The solid-state fermented mycelia refer to edible fungus mycelia produced by solid-state fermentation, and the secondary fermented mycelia refer to edible fungus mycelia produced by secondary fermentation. To avoid overly long prefixes, the edible fungus mycelia produced by solid-state fermentation and the edible fungus mycelia produced by secondary fermentation are referred to as solid-state fermented mycelia and secondary fermented mycelia in the following content.

Edible fungi refer to edible mushrooms, including three parts: mycelium, fruiting bodies and spores. Common edible fungi include: *Lentinus edodes, Volvariella volvacea, Agaricus bisporus, Auricularia auricula, Tremella fuciformis, Hericium erinaceus, Dictyophora indusiata, Tricholoma matsutake, Tricholoma mongolicum, Russula vinosa, Ganoderma lucidum, Cordyceps sinensis, Tuber melanosporum, Pleurotus nebrodensis* and *Boletus edulis.* Edible fungus mycelium is formed by the germination and reproduction of edible fungus spores.

The edible fungi and edible fungus mycelia used in the present disclosure are available to the public through conventional channels, and are not limited to a specific type of edible fungus mycelium.

Janus, that is, Janus in Roman mythology, and refers to a concept of "two-sidedness"; and Janus paper is defined as paper with different properties on its two sides. In the present disclosure, the Janus paper mainly includes two parts, which are solid-state fermented edible fungus mycelia and secondary fermented edible fungus mycelia. Solid-state fermentation refers to a process in which edible fungus mycelia are cultivated using a solid culture medium in the absence of or with a minimal amount of free water, enabling the edible fungus mycelia to undergo biological reactions, grow and reproduce. In other words, solid-state fermentation is a process of cultivating edible fungus mycelia using a solid culture medium. Solid culture media often use crops such as wheat, corn, and soybeans, as well as processing by-products thereof including wheat bran, corn flour, and soybean meal as main raw materials. These substances are rich in carbon sources, nitrogen sources, and other nutrients, and can provide the necessary nutrition for the growth of mycelia. Secondary fermentation uses edible fungus mycelia as raw materials and is carried out with Trichoderma fermentation. In the fermentation process, chitin in the mycelia is separated from substances such as cellulose and protein; and the fermentation broth is centrifuged and washed with water, and the final precipitate obtained is a composite of chitin and glucan. In other words, secondary fermentation is a process in which edible fungus mycelia are decomposed by another type of microorganism.

To remove moisture from the mycelia and achieve the objective of inactivation simultaneously, the edible fungus mycelia from solid-state fermentation and the edible fungus mycelia from secondary fermentation are separately subjected to drying treatment in this step. The drying methods can be high-temperature heating drying, vacuum drying, etc., and the drying time can be adjusted according to actual conditions, with the objective of achieving a good inactivation effect while retaining the fiber properties of the mycelia.

Further, in one embodiment of the present disclosure, electric blast is used for drying the mycelia, specifically: the solid-state fermented mycelia and secondary fermented mycelia were placed in electric blast drying ovens and treated at a temperature of 45-65°C for 6-12 hours.

In step 2, fibrillation of mycelia: the inactivated solid-state fermented mycelia and inactivated secondary fermented mycelia obtained in step 1 were placed in crushers and crushed; the crushed mycelia were mixed with water to form Suspension A and Suspension B, in which Suspension A was a suspension included by inactivated solid-state fermented mycelia and water, and Suspension B was a suspension included by inactivated secondary fermented mycelia and water; Suspension A and Suspension B were ground; and the ground Suspension A and Suspension B were subjected to centrifugation using centrifuges, and fibrillated solid-state fermented mycelia and fibrillated secondary fermented mycelia were separated.

Fibrillation refers to a phenomenon that the fiber cell wall is fluffed, teared, and filamented after the pulp is beaten in pulping and papermaking production. In the papermaking process, fiber fibrillation is of great importance. When fibers are fibrillated, the specific surface area will increase. After beating and other treatments, the fine fibers on the surface of the originally smooth fibers will be divided into filaments and fluffed, just like a branch becomes more fluffy. Such fibers can be better intertwined during papermaking, thereby improving the strength, uniformity and tightness of the paper. Paper made from non-fibrillated fibers has poor performance and low tensile strength. This is because the bonding between fibers is not tight enough; and when stretched, the fibers are easy to slide with each other, leading to paper breakage. Non-fibrillated fibers are distributed randomly in the paper, which tends to cause local fiber accumulation or looseness, resulting in uneven paper texture. In contrast, fibrillated fibers can interweave and entangle with each other more effectively. When the paper is subjected to tensile force, these tightly interwoven fibers can bear external forces collectively, preventing the paper from breaking easily and improving its tensile strength. Additionally, due to the more uniform distribution of fibrillated fibers, the paper can achieve consistent texture both in the thickness direction and the planar direction.

In this instance, the pulp is mycelial pulp. By crushing and grinding the inactivated mycelia, the mycelia can be further refined to achieve fibrillation. Successfully fibrillated mycelial fibers exhibit obvious fibrillation phenomena; and for instance, originally smooth and intact fibers, after fibrillation, resemble a brush with numerous fine branches on the surface thereof. These fine branches can enhance the interweaving ability between fibers. The fiber length remains basically unchanged, but the width may increase due to fibrillation. This is because when fibers are subjected to mechanical actions (such as crushing and grinding), the internal fine fiber structure is unfolded and extends outward from the fiber main body, making the fibers appear "bulkier" overall. Subsequently, the fibrillated mycelial suspension is subjected to centrifugation using a centrifuge to separate fibrillated solid-state fermented mycelia and fibrillated secondary fermented mycelia.

In one embodiment, specific steps of fibrillation of mycelia in step 2 are as follows: the inactivated solid-state fermented mycelia and inactivated secondary fermented mycelia obtained in step 1 were placed in crushers and treated at a power of 3000 W for 1-4 minutes; the two types of crushed mycelia were mixed with purified water to form inactivated solid-state fermented mycelial Suspension A and inactivated secondary fermented mycelial Suspension B, with the mycelium concentration in both Suspension A and Suspension B adjusted to 30-50 g/100 mL; disc refiners were used to grind Suspension A and Suspension B, with a rotation speed set to 3000-10000 r/min and a treatment time set to 20-60 minutes; and centrifuges were used to centrifuge the ground Suspension A and Suspension B, at a rotation speed of 6500-8500 r/min for 10-20 minutes, to obtain fibrillated solid-state fermented mycelia and fibrillated secondary fermented mycelia.

After step 2, the method further includes screening the obtained fibrillated solid-state fermented mycelia and fibrillated secondary fermented mycelia: a laser particle size analyzer was used to screen the fibrillated solid-state fermented mycelia and fibrillated secondary fermented mycelia, ensuring the particle sizes are no more than 200 µm; and a fiber morphology analyzer was used to screen the fibrillated solid-state fermented mycelia and fibrillated secondary fermented mycelia, ensuring the fibrillation rates are no less than 50%.

The laser particle size analyzer can measure particle size distribution data. The fiber morphology analyzer can observe and analyze fiber morphological characteristics, such as length, width, fine fiber content, fiber curl and kink, etc. The fiber morphology analyzer can be used to identify the quality of fiber raw materials, guide the control of beating conditions, predict pulp applications, and identify the quality of pulping processes.

Fibrillation rate is an indicator used to measure the degree of fiber fibrillation, such as fibril separation and fuzzing after fibers are subjected to mechanical or chemical actions during pulping, and it is mainly used in the papermaking industry. The determination of fiber fibrillation rate can be carried out in accordance with the national standard GB/T 22836-2008 Determination of Fibrillation Rate of Pulp Fibers, or calculated by counting the number of fibrillated fibers and the total number of fibers using the formula: fibrillation rate = (number of fibrillated fibers / total number of fibers) × 100%.

It is to be additionally noted that the examples of the present disclosure provide some specific operating instruments (such as disc refiners), operating values (such as treatment at a power of 3000 W for 1-4 minutes), and screening thresholds (such as a particle size of no more than 200 µm). These data are optimal settings determined based on experience and multiple experiments. It can be understood that, within the overall technical framework of this application, those skilled in the art can flexibly select existing technologies in actual operations to achieve the same operating effects, or flexibly adjust these values to achieve the best operating conditions and screening conditions. For example, appropriate crushing and grinding methods and durations can be selected according to the scale of the paper to be prepared and the actual condition of the mycelia, and higher or lower screening conditions can be adopted according to the required standards. The same logic applies to the following operating steps, which will not be repeated.

In step 3, purification of solid-state fermented mycelia: the fibrillated solid-state fermented mycelia obtained in step 2 were mixed with water to form Suspension C; Suspension C was subjected to centrifugation using a centrifuge to obtain solid-state fermented mycelial precipitate; the solid-state fermented mycelial precipitate was placed in a sodium hydroxide solution, and a mixture of the solid-state fermented mycelial precipitate and sodium hydroxide was placed in a constant-temperature water bath and stirred; and pure water was repeatedly added to the mixture and centrifugation was performed to obtain a precipitate as purified solid-state fermented mycelia.

An objective of sodium hydroxide in this step is to remove proteins and fats from the solid-state fermented mycelia. Proteins and fats have adverse effects on papermaking processes and paper properties. For example, proteins and fats are not easily dispersed or dissolved during pulping, and are tend to form aggregates, which interfere with the pulping process and reduce the uniformity of the pulp. The strength of paper mainly comes from the hydrogen bonding between fibers; and as non-fibrous components, proteins and fats interfere with the close contact between fibers and the formation of hydrogen bonds, thereby affecting the strength of the paper. In addition, proteins and fats are prone to deterioration during paper storage, which impairs the quality of the paper.

In the papermaking process, non-fibrous components such as proteins and fats in the mycelia can be effectively removed by adjusting the concentration of sodium hydroxide and the treatment time, thereby improving the uniformity of the pulp and the properties of the paper. A concentration of the sodium hydroxide solution can be adjusted according to the tolerance of the solid-state fermented mycelia. Preferably, a concentration of sodium hydroxide solution ranges from 0.5 mol/L to 1.5 mol/L, and a concentration of solid-state fermented mycelial precipitate in the sodium hydroxide solution ranges from 30 g/100 mL to 50 g/100 mL.

A constant-temperature water bath can maintain the mixture at a certain temperature, promoting the treatment effect of sodium hydroxide.

Repeated addition of pure water and centrifugation can remove impurities and residual sodium hydroxide solution from the solid-state fermented mycelia, and the number of repetitions can be determined according to the cleaning effect.

In one embodiment, specific steps for the purification of solid-state fermented mycelia in step 3 are as follows: the fibrillated solid-state fermented mycelia obtained in step 2 were mixed with purified water to form Suspension C with a concentration of 30-50 g/100 mL; the suspension was placed in a constant-temperature water bath at 70-85°C and stirred with an electric stirrer at a rotation speed of 200-400 r/min for 30-60 minutes; suspension C was centrifuged using a centrifuge at a rotation speed of 6500-8500 r/min for 15-20 minutes; the obtained solid-state fermented mycelial precipitate was placed in a 0.5-1.5 mol/L sodium hydroxide solution, with a concentration of the precipitate in the solution being 30-50 g/100 mL; the mixture of the solid-state fermented mycelial precipitate and sodium hydroxide was placed in a constant-temperature water bath at 50-65°C and stirred with an electric stirrer at a rotation speed of 220-420 r/min for 1.5-3.5 hours; purified water with a volume of 2.5-3.5 times that of the mixture was added to the mixture, and centrifugation was performed at a rotation speed of 6500-8500 r/min for 15-20 minutes; and the precipitate was collected, and the operations of adding water and centrifugation were repeated 3-4 times to obtain the final precipitate as purified solid-state fermented mycelia.

Through the above operations, the fibrillated solid-state fermented mycelia can be purified to obtain solid-state fermented mycelia with high purity.

After step 3, the method further includes screening the obtained purified solid-state fermented mycelia, specifically: a pH meter was used to screen the purified solid-state fermented mycelia to ensure the pH value is 7.5±0.5; a laser confocal fluorescence microscope was used to screen the purified solid-state fermented mycelia to ensure the average length is no more than 20 µm; and an oven was used to screen the purified solid-state fermented mycelia to ensure the heat resistance is no less than 180°C.

A pH meter, also known as an acid-base detector, can measure the pH value of the measured object.

A laser confocal fluorescence microscope can observe and measure the length of mycelia; and by recording the mycelial length data, mycelia that meet the length requirement can be screened out.

The measurement of heat resistance is carried out by placing a certain amount of purified solid-state fermented mycelia in an oven, setting a temperature to 180°C and maintaining the temperature for a certain period of time, observing and recording changes of the mycelia during heating, such as color and morphology, and screening out mycelia that can still maintain a good morphology after heating.

The quality of the purified solid-state fermented mycelia can be further ensured by screening.

In step 4, gelation of secondary fermented mycelia: the fibrillated secondary fermented mycelia obtained in step 2 were mixed with water to form Suspension D; Suspension D was treated with a homogenizer to make Suspension D more uniform; Suspension D was placed in a constant-temperature water bath, EGCG was added, and the mixture was stirred; and after stirring, Suspension D was subjected to centrifugation using a centrifuge to obtain a precipitate as gelled secondary fermented mycelia.

EGCG is the most effective active component in tea polyphenols and has strong antioxidant properties. Adding EGCG to the fibrillated secondary fermented mycelia can bridge the hydroxyl groups of the mycelia, which helps the secondary fermented mycelia to form a three-dimensional network gel structure. An addition amount of EGCG can be adjusted according to specific requirements and experimental conditions to achieve the optimal gelation effect and functional properties.

In one embodiment of the present example, a weight ratio of EGCG to secondary fermented mycelia ranges from 1:50 to 1:6, and a weight ratio of EGCG to a total weight of mycelia (i.e., a total weight of secondary fermented mycelia and solid-state fermented mycelia) ranges from 1:100 to 1:12.

In one embodiment, the gelation of secondary fermented mycelia in step 4 includes the following steps: the fibrillated secondary fermented mycelia obtained in step 2 were mixed with purified water to form Suspension D with a concentration of 30-50 g/100 mL; the suspension was treated with a homogenizer at a rotation speed of 16900-21900 r/min for 1-4 minutes; Suspension D was placed in a constant-temperature water bath at 75-95°C, EGCG was added to Suspension D, with a concentration of EGCG in Suspension D being 1-5 g/100 mL, and the mixture was stirred with an electric stirrer at a rotation speed of 200-400 r/min for 30-60 minutes; and Suspension D was centrifuged using a centrifuge at a rotation speed of 6500-8500 r/min for 15-20 minutes to obtain the precipitate as gelled secondary fermented mycelia.

Further, after Step 4, the method further includes screening the obtained gelled secondary fermented mycelia, specifically: a scanning electron microscope was used to screen the gelled mycelia to ensure the gelled secondary fermented mycelia exhibit a network structure; a rheometer was used to screen the gelled mycelia to ensure the viscous modulus is no less than 35 Pa and the elastic modulus is no less than 40 Pa; and a texture analyzer was used to screen the gelled mycelia to ensure the hardness is no less than 350 g/cm².

In step 5, preparation of Janus paper: the purified solid-state fermented mycelia obtained in step 3 and the gelled secondary fermented mycelia obtained in step 4 were mixed with water to form Suspension E and Suspension F, in which Suspension E was a suspension of purified solid-state fermented mycelia, and Suspension F was a suspension of gelled secondary fermented mycelia. Suspension E and Suspension F were sequentially poured into a paper-forming mold to form mycelial filter cakes. The two overlapping mycelial filter cakes were pressed using a paper flattener, and a weight with uniform mass distribution was pressed on the forming mold with the two mycelial filter cakes. The forming mold with the two mycelial filter cakes and the weight was placed in an oven for drying treatment; and after drying, the forming mold with the two types of mycelia and the weight was taken out and cooled at room temperature; and after cooling, the weight and the mold were removed, and Janus paper prepared from edible fungus mycelia was obtained.

In the present disclosure, the Janus paper includes two sides: one side is included by solid-state fermented mycelia, and the other side is included by secondary fermented mycelia. The paper with two layers is formed by stacking, and each layer has unique properties. The paper surface included by purified solid-state fermented mycelia is relatively rough, which endows it with a certain oil adsorption capacity and lipophilic (oil-absorbing) properties; while the paper surface included by gelled secondary fermented mycelia is smooth and dense after gelation treatment, has oil-repellent capacity, can prevent oil penetration, and exhibits oil-repellent (oil-impermeable) properties.

Firstly, the suspension of purified solid-state fermented mycelia was poured into a paper-forming mold, after a filter cake was formed, the suspension of gelled secondary fermented mycelia was poured into the mold to form a second filter cake, resulting in a mycelial filter cake with a two-layer structure, and each layer included a different type of mycelia. Subsequently, the two overlapping mycelial filter cakes were initially pressed using a paper flattener to remove excess water and make the filter cakes more compact; and a weight was pressed on the mold to further increase the density and strength of the paper. The mold and the weight were placed in an oven for drying treatment. The temperature and time of the oven could be adjusted according to the properties of the mycelia and the desired drying degree of the paper. After drying, the mold and the weight were cooled at room temperature to prevent the paper from deforming or breaking due to temperature changes when taken out. The weight and the mold were removed, and the complete Janus paper was obtained.

In one embodiment, specific steps for the preparation of Janus paper in step 5 are as follows: the purified solid-state fermented mycelia obtained in step 3 and the gelled secondary fermented mycelia obtained in step 4 were mixed with purified water to form Suspension E and Suspension F with a concentration of 0.5-1.5 g/100 mL. Suspension E and F were sequentially poured into a rectangular mold with a width of 18 cm and a length of 29 cm to form rectangular mycelial filter cakes with a thickness of 90-240 g/m²; the two overlapping rectangular mycelial filter cakes were pressed using a paper flattener for 15-30 minutes; a 5 kg weight with uniform mass distribution was pressed on the rectangular paper mold with the two mycelial filter cakes, and the rectangular paper mold with the two mycelial filter cakes and the weight was placed in an oven, with the temperature set to 110-130°C for drying treatment for 2-4 hours; and the paper mold with the two types of mycelia and the weight was taken out and placed at room temperature for 8-12 hours, after that, the weight and the mold were removed, and the Janus paper prepared from edible fungus mycelia was obtained.

Further, after step 5, the method further includes screening the edible fungus mycelium Janus paper that has no shrinkage, no deformation, uniform thickness, uniform and flat paper surface, and good symmetry between the solid-state fermented mycelium surface and the secondary fermented mycelium surface, specifically: a solid-state fermented mycelium paper was screened to ensure the paper has a relatively rough surface and an oil-phase interface contact angle thereof does not exceed 75°; an oil-phase interface contact angle of a secondary fermented mycelium paper surface was screened to ensure the angle ranges from 90° to 180°; and Janus paper was screened to ensure the compressive strength is no less than 3 MPa, transverse tear strength no less than 1500 mN, longitudinal tear strength no less than 1200 mN, transverse tensile strength no less than 4.5 kN/m, longitudinal tensile strength no less than 5.5 kN/m, and elongation at break no less than 15%. The oil-phase interface contact angle could be measured using a contact angle meter; the compressive strength could be obtained using a pressure testing machine; and the tear strength and tensile strength could be tested using a tensile testing machine.

In the present disclosure, components of Janus paper include: solid-state fermented mycelia and secondary fermented mycelia obtained by solid-state fermentation and secondary fermentation of edible fungus mycelia, and EGCG. A mass ratio of solid-state fermented mycelia to secondary fermented mycelia is generally 1:1; a weight ratio of epigallocatechin gallate to secondary fermented mycelia ranges from 1:50 to 1:6; and a weight ratio of EGCG to the total weight of mycelia ranges from 1:100 to 1:12.

The example of the present disclosure also provides a Janus paper, which is prepared by the method for preparing Janus paper by mycelia.

The Janus paper in the example of the present disclosure includes two surfaces, one surface is a solid fermentation mycelium paper surface, and the other surface is a secondary fermentation mycelium paper surface.

In the present disclosure, the mechanism by which Janus paper is prepared from edible fungus mycelia is as follows: Janus paper refers to paper with different properties on its two sides, and the properties are achieved in the present disclosure by utilizing the distinct properties of solid-state fermented mycelia and secondary fermented mycelia. In terms of chemical composition, main components of both solid-state fermented mycelia and secondary fermented mycelia are glucan, chitin, protein, and other substances. Non-fibrous components such as protein in the mycelia are removed through purification treatment, while chitin and glucan are retained. In the treatment process, a composite structure is formed by chitin and glucan in the mycelia: chitin plays a role as a framework, and glucan acts as a filler that fills into the framework formed by chitin, enhancing the toughness and strength of chitin. This composite structure endows the mycelia with material properties suitable for papermaking. Furthermore, the fibrillation rate of the mycelia is increased through pre-grinding treatment, more hydroxyl groups on the mycelia are exposed, and the efficiency of hydrogen bonding between mycelia via hydroxyl groups is improved. These factors collectively promote paper formation and endow the solid-state fermented mycelium paper with flexibility, hydrophobicity, and oil absorbency. In addition, EGCG is a natural antioxidant derived from green tea and has the function of bridging hydroxyl groups of mycelia. EGCG interacts with the hydroxyl groups of mycelia through hydrogen bonds, which consumes the number of hydroxyl groups on the mycelial surface. This helps reduce the hydrophilicity of the mycelia and improve the lipophilicity of the mycelia. Meanwhile, with the help of the bridging effect of EGCG, the formation of a three-dimensional network gel structure by secondary fermented mycelia is facilitated. This structure not only enhances properties of the mycelium paper such as compressive strength, tensile strength, and tear strength but also fixes oil in the mycelial gel network structure, thereby achieving the effect of oil impermeability. Therefore, the paper prepared by mycelia can simultaneously achieve multiple advantages such as safety, material properties and double-sidedness of paper.

### Example 2

The technical solution of the present disclosure will be further described in detail with reference to a specific example and the accompanying drawings, and it is to be understood that the following examples are merely for explaining the present disclosure, and are not intended to limit the present disclosure.

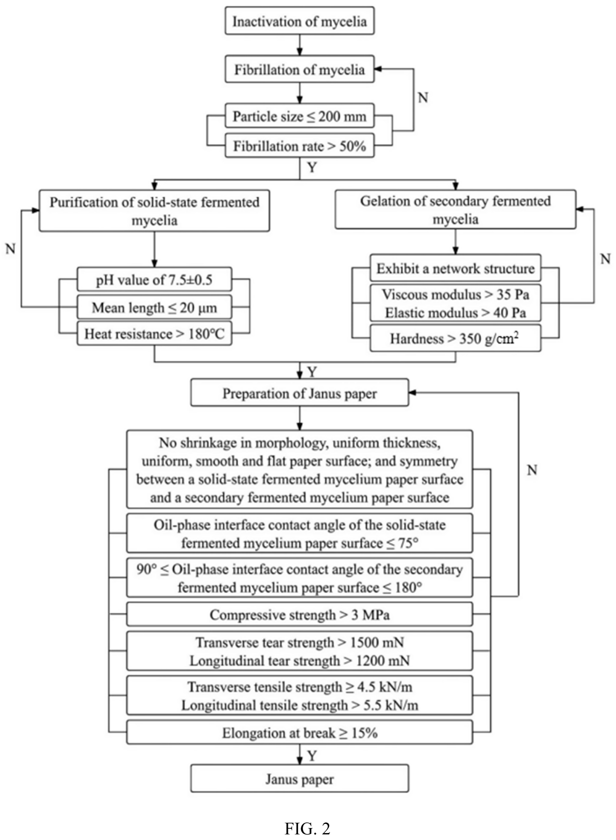

Referring to FIG. 2, FIG. 2 is a schematic flow diagram in an implementation scenario according to an example of the present disclosure. As shown in FIG. 2, a method for preparing Janus paper by mycelia in the example of the present disclosure includes the following steps.

In step 1, inactivation of mycelia:
The edible fungus mycelia from solid-state fermentation and the edible fungus mycelia from secondary fermentation were placed in electric blast drying ovens and treated at a temperature of 45°C for 12 hours to obtain inactivated solid-state fermented mycelia and inactivated secondary fermented mycelia.

In step 2, fibrillation of mycelia:
The inactivated solid-state fermented mycelia and inactivated secondary fermented mycelia obtained in step 1 were placed in crushers and treated at a power of 3000 W for 3 minutes. The two types of crushed mycelia were mixed with purified water to form an inactivated solid-state fermented mycelial suspension and an inactivated secondary fermented mycelial suspension, with the mycelium concentration adjusted to 33 g/100 mL. A disc refiner was used to grind the two mycelial suspensions, with a rotation speed set to 9990 r/min and the treatment time set to 35 minutes. A centrifuge was used to centrifuge the two ground mycelial suspensions at a rotation speed of 7500 r/min for 15 minutes to obtain fibrillated solid-state fermented mycelia and fibrillated secondary fermented mycelia.

A laser particle size analyzer was used to screen the fibrillated solid-state fermented mycelia and fibrillated secondary fermented mycelia to ensure the particle sizes are no more than 200 µm. A fiber morphology analyzer was used to screen the fibrillated solid-state fermented mycelia and fibrillated secondary fermented mycelia to ensure the fibrillation rates are no less than 50%.

For mycelia that meet the screening conditions, the next step is carried out; otherwise, this step is repeated to re-fibrillate the mycelia until the screening conditions are met or the mycelia are discarded. The same screening logic applies to the following steps and will not be repeated.

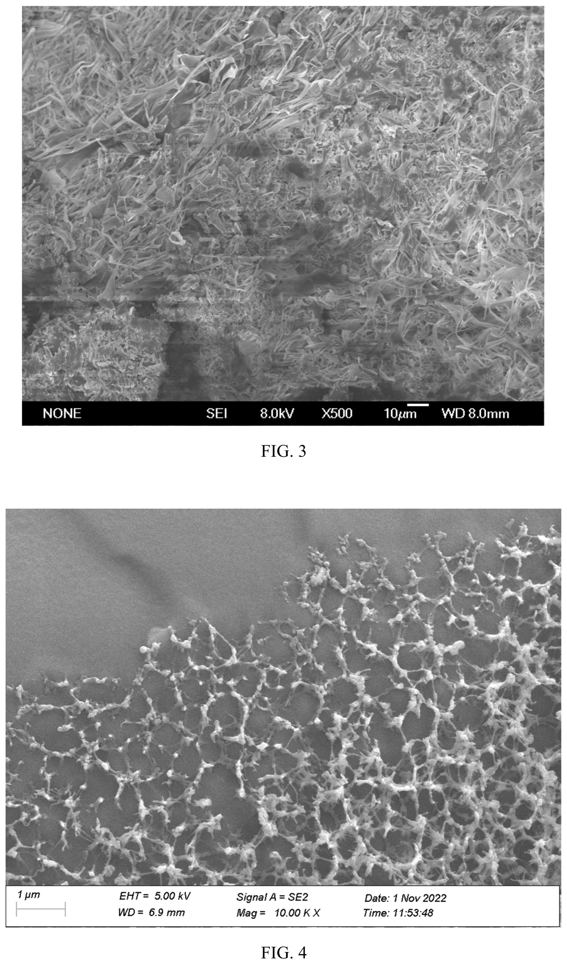

In step 3, purification of solid-state fermented mycelia:
The fibrillated solid-state fermented mycelia obtained in step 2 were mixed with purified water to form a fibrillated solid-state fermented mycelial suspension with a concentration of 33 g/100 mL. The suspension was placed in a constant-temperature water bath at 85°C and stirred with an electric stirrer at a rotation speed of 200 r/min for 40 minutes. A centrifuge was used to centrifuge the fibrillated solid-state fermented mycelial suspension at a rotation speed of 7500 r/min for 15 minutes. The obtained mycelial precipitate was placed in a 1 mol/L sodium hydroxide solution, with a concentration of the mycelial precipitate in the solution being 33 g/100 mL. The mixture was placed in a constant-temperature water bath at 60°C and stirred with an electric stirrer at a rotation speed of 300 r/min for 3 hours. Purified water with a volume three times that of the mixture was added to the mixture, and centrifugation was performed at a rotation speed of 7500 r/min for 15 minutes. The precipitate was collected, and the operations of adding water and centrifugation were repeated three times to obtain a final precipitate as purified solid-state fermented mycelia. Referring to FIG. 3, FIG. 3 shows a microscopic morphology of solid-state fermented mycelia.

A pH meter was used to screen the purified solid-state fermented mycelia to ensure the pH value is 7.5+0.5. A laser confocal fluorescence microscope was used to screen the purified solid-state fermented mycelia to ensure the average length is no more than 20 µm. An oven was used to screen the purified solid-state fermented mycelia to ensure the heat resistance is no less than 180°C.

In step 4, gelation of secondary fermented mycelia:
The fibrillated secondary fermented mycelia obtained in step 2 were mixed with purified water to form a fibrillated secondary fermented mycelial suspension with a concentration of 33 g/100 mL. A homogenizer was used to treat the suspension at a rotation speed of 19990 r/min for 3 minutes. The fibrillated secondary fermented mycelial suspension was placed in a constant-temperature water bath at 90°C, and EGCG was added to the suspension, with a concentration of EGCG in the suspension adjusted to 3 g/100 mL. The mixture was stirred with an electric stirrer at a rotation speed of 240 r/min for 45 minutes. A centrifuge was used to centrifuge the fibrillated secondary fermented mycelial suspension at a rotation speed of 7500 r/min for 15 minutes to obtain a precipitate as gelled secondary fermented mycelia. Referring to FIG. 4, FIG. 4 shows a microscopic morphology of a network structure of gelled secondary fermented mycelia.

A scanning electron microscope was used to screen the gelled secondary fermented mycelia to ensure the gelled secondary fermented mycelia exhibit a network structure. A rheometer was used to screen the gelled secondary fermented mycelia to ensure the viscous modulus is no less than 35 Pa and the elastic modulus is no less than 40 Pa. A texture analyzer was used to screen the gelled secondary fermented mycelia to ensure the hardness is no less than 350 g/cm².

In step 5, preparation of Janus paper:
The purified solid-state fermented mycelia obtained in step 3 and the gelled secondary fermented mycelia obtained in step 4 were mixed with purified water to form a purified solid-state fermented mycelial suspension and a gelled secondary fermented mycelial suspension, both with a concentration of 1 g/100 mL. The two mycelial suspensions were sequentially poured into a rectangular mold with a width of 18 cm and a length of 29 cm to form rectangular mycelial filter cakes with a thickness of 125 g/m². A paper flattener was used to press the two overlapping rectangular mycelial filter cakes for 30 minutes. A 5 kg weight with uniform mass distribution was pressed on the rectangular paper mold with the two mycelial filter cakes, and the mold with the filter cakes and the weight was placed in an oven, with a temperature set to 115°C for drying treatment for 3 hours. The paper mold with the two types of mycelia and the weight was taken out and placed at room temperature for 12 hours. After that, the weight and the mold were removed, and Janus paper prepared from edible fungus mycelia was obtained.

Janus paper was screened to ensure Janus paper has no shrinkage, no deformation, uniform thickness, a uniform, smooth, and flat paper surface, and good symmetry between the solid-state fermented mycelium surface and the secondary fermented mycelium surface. The solid-state fermented mycelium paper surface was screened to ensure the oil-phase interface contact angle is no more than 75°. The secondary fermented mycelium paper surface was screened to ensure the oil-phase interface contact angle ranges from 90° to 180°. The Janus paper was further screened to ensure the compressive strength is no less than 3 MPa, transverse tear strength no less than 1500 mN, longitudinal tear strength no less than 1200 mN, transverse tensile strength no less than 4.5 kN/m, longitudinal tensile strength no less than 5.5 kN/m, and elongation at break no less than 15%.

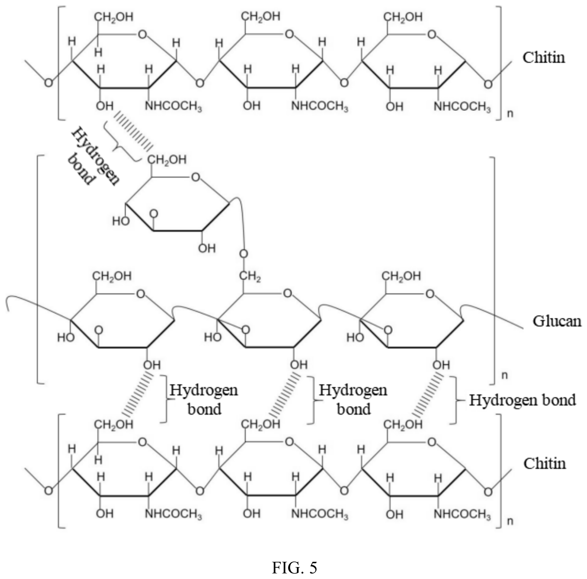

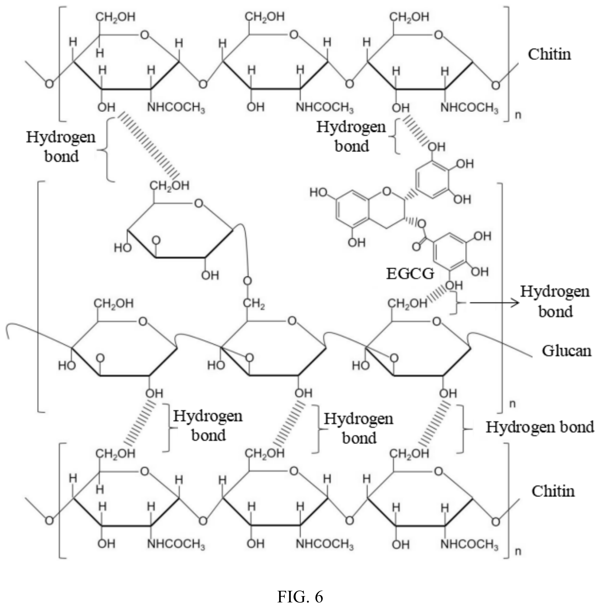

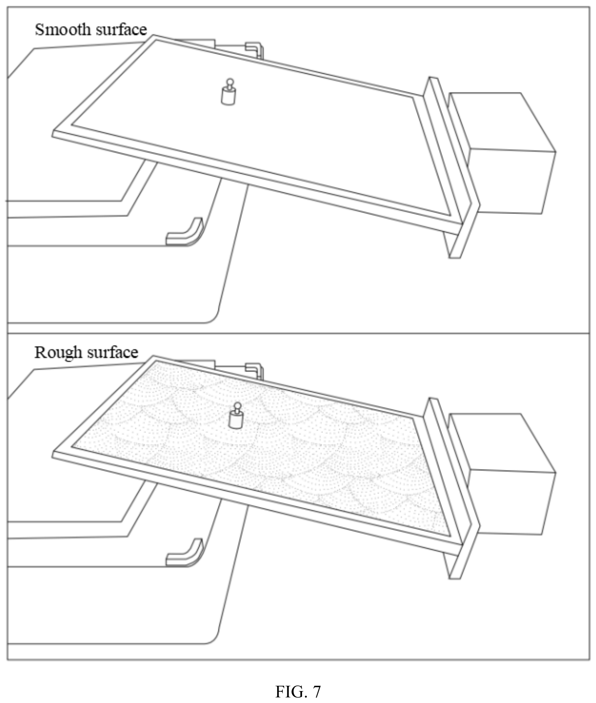

After the implementation of the above steps, the Janus paper prepared from edible fungus mycelia is processed and completed. The molecular structure of the solid-state fermented mycelium paper surface is shown in FIG. 5, the molecular structure of the secondary fermented mycelium paper surface is shown in FIG. 6, and the morphology of the prepared Janus paper is shown in FIG. 7.

The steps in the method of the examples of the present disclosure can be sequentially adjusted, merged and deleted according to actual needs. In the solutions of the present disclosure, the description of each example has different emphasis, and for parts not described or described in detail in a certain example, please refer to the related description of other examples. The technical features of the technical solutions of the present disclosure can be arbitrarily combined, and all possible combinations of the technical features in the examples have not been described in order to make the description concise, but as long as there is no contradiction between the combinations of the technical features, all such combinations shall be considered to fall within the scope of the present disclosure.

The above are only preferred examples of the present disclosure, it is to be pointed out that for those ordinary skilled in the art, several improvements and embellishments can be made without departing from the principle of the present disclosure, and these improvements and embellishments are also regarded as the protection scope of the present disclosure.

## Claims

1. A method for preparing Janus paper by mycelia, wherein the mycelium is edible fungus mycelium, and the method comprises the steps of:
step 1, inactivation of mycelia: placing solid-state fermented mycelia and secondary fermented mycelia in drying ovens for drying to obtain inactivated solid-state fermented mycelia and inactivated secondary fermented mycelia; and the solid-state fermented mycelia referring to edible fungus mycelia cultured via solid-state fermentation, the secondary fermented mycelia referring to edible fungus mycelia processed via secondary fermentation, the solid-state fermentation referring to a process of culturing edible fungus mycelia using solid media, the secondary fermentation referring to a process of decomposing edible fungus mycelia using another microorganism, and the edible fungi referring to edible mushrooms;
step 2, fibrillation of mycelia: crushing the inactivated solid-state fermented mycelia and inactivated secondary fermented mycelia obtained in step 1 in crushers; preparing the crushed mycelia into Suspension A and Suspension B with water, and grinding Suspension A and Suspension B; and performing centrifugation on the ground Suspension A and Suspension B using centrifuges to separate fibrillated solid-state fermented mycelia and fibrillated secondary fermented mycelia;
step 3, purification of solid-state fermented mycelia: preparing the fibrillated solid-state fermented mycelia obtained in step 2 into Suspension C with water; performing centrifugation on Suspension C using a centrifuge to obtain solid-state fermented mycelial precipitate; placing the solid-state fermented mycelial precipitate in a sodium hydroxide solution, and stirring a mixture of the precipitate and sodium hydroxide in a constant-temperature water bath; and repeatedly adding water to the mixture and performing centrifugation to obtain a precipitate as purified solid-state fermented mycelia;
step 4, gelation of secondary fermented mycelia: preparing the fibrillated secondary fermented mycelia obtained in step 2 into Suspension D with water; treating Suspension D using a homogenizer to make Suspension D more uniform; placing Suspension D in a constant-temperature water bath, adding epigallocatechin gallate (EGCG), and stirring; and performing centrifugation on Suspension D using a centrifuge after stirring to obtain a precipitate as gelled secondary fermented mycelia; and
step 5, preparation of Janus paper: preparing the purified solid-state fermented mycelia obtained in step 3 and the gelled secondary fermented mycelia obtained in step 4 into Suspension E and Suspension F with water; pouring Suspension E and Suspension F into a paper forming mold in sequence to form mycelial filter cakes; pressing the two overlapping mycelial filter cakes using a paper flattening machine; applying a uniformly distributed weight on the forming mold with the two filter cakes; placing the forming mold with the filter cakes and the weight into an oven for drying; taking out the mold with the two types of mycelia and the weight after drying, and cooling the mold with the two types of mycelia and the weight at room temperature; and removing the weight and the mold after cooling to obtain Janus paper prepared from edible fungus mycelia.

2. The method for preparing Janus paper by mycelia according to claim 1, wherein in step 3, a concentration of sodium hydroxide solution ranges from 0.5 mol/L to 1.5 mol/L, and a concentration of solid-state fermented mycelial precipitate in the sodium hydroxide solution ranges from 30 g/100 mL to 50 g/100 mL.

3. The method for preparing Janus paper by mycelia according to claim 1, wherein in step 3, the stirring mixtures of the precipitates and sodium hydroxide in constant-temperature water baths; and repeatedly adding water to the mixtures and performing centrifugation to obtain precipitates as purified solid-state fermented mycelia comprises the steps of:
placing the mixture in a constant-temperature water bath at 50-65°C, and stirring the mixture with an electric stirrer at a rotation speed of 220-420 r/min for 1.5-3.5 hours; and
adding purified water of 2.5-3.5 times the volume to the mixture, performing centrifugation at a rotation speed of 6500-8500 r/min for 15-20 minutes, collecting the precipitate, and repeating operations of adding water to the precipitate and centrifuging for 3-4 times to obtain the precipitate as purified solid-state fermented mycelia.

4. The method for preparing Janus paper by mycelia according to claim 1, wherein in step 4, a weight ratio of EGCG to the secondary fermented mycelia ranges from 1:50 to 1:6, a weight ratio of EGCG to a total weight of mycelia ranges from 1:100 to 1:12, and the total weight of mycelia refers to a total weight of the secondary fermented mycelia and the solid-state fermented mycelia.

5. The method for producing Janus paper from mycelium according to claim 1, wherein step 4 comprises the steps of:
preparing the fibrillated secondary fermented mycelia obtained in step 2 into Suspension D with a concentration of 30-50 g/100 mL using purified water, and treating Suspension D with a homogenizer at a rotation speed of 16900-21900 r/min for 1-4 minutes;
placing Suspension D in a constant-temperature water bath at 75-95°C, adding EGCG to Suspension D, with a concentration of EGCG in Suspension D being 1-5 g/100 mL, and stirring with an electric stirrer at a rotation speed of 200-400 r/min for 30-60 minutes; and
performing centrifugation on Suspension D using a centrifuge, setting a rotation speed to 6500-8500 r/min and centrifuging for 15-20 minutes to obtain the precipitate as gelled secondary fermented mycelia.

6. The method of preparing Janus paper by mycelium according to claim 1, wherein after step 2, the method further comprises the steps of:
screening particle sizes of fibrillated solid-state fermented mycelia and fibrillated secondary fermented mycelia using a laser particle size analyzer, ensuring both are no more than 200 µm; and screening fibrillation rates of fibrillated solid-state fermented mycelia and fibrillated secondary fermented mycelia using a fiber morphology analyzer, ensuring both are no less than 50%.

7. The method of preparing Janus paper by mycelium according to claim 1, wherein after step 3, the method further comprises the steps of:
screening the pH value of purified solid-state fermented mycelia using a pH meter, ensuring the pH is 7.5±0.5; screening the average length of purified solid-state fermented mycelia using a laser confocal fluorescence microscope, ensuring the average length is no more than 20 µm; and screening the heat resistance of purified solid-state fermented mycelia using an oven, ensuring the heat resistance is no less than 180°C.

8. The method of preparing Janus paper by mycelium according to claim 1, wherein after step 4, the method further comprises the steps of:
screening the gelled secondary fermented mycelia using a scanning electron microscope to ensure the gelled secondary fermented mycelia exhibit a network structure; screening the gelled secondary fermented mycelia using a rheometer to ensure the viscous modulus is no less than 35 Pa and elastic modulus is no less than 40 Pa; and screening the gelled secondary fermented mycelia using a texture analyzer to ensure the hardness is no less than 350 g/cm².

9. The method of preparing Janus paper by mycelium according to claim 1, wherein after step 5, the method further comprises the steps of:
screening an oil-phase interface contact angle of a solid-state fermented mycelium paper surface to ensure the angle is no more than 75°; screening an oil-phase interface contact angle of a secondary fermented mycelium paper surface to ensure the angle ranges from 90° to 180°; and screening Janus paper to ensure a compressive strength is no less than 3 MPa, transverse tear strength no less than 1500 mN, longitudinal tear strength no less than 1200 mN, transverse tensile strength no less than 4.5 kN/m, longitudinal tensile strength no less than 5.5 kN/m, and elongation at break no less than 15%.

10. A Janus paper sheet, wherein the Janus paper is prepared by the method for preparing Janus paper by mycelia according to any one of claims 1 to 9.
